# EUROPEAN PATENT APPLICATION

(11) **EP 3 189 833 A1**
(43) Date of publication of application: **12.07.2017**
(21) Application number: 17153266.6
(22) Date of filing: 21.11.2007
(51) Int. Cl.: A61K 9/70, A61K 47/10, A61K 47/42, A61L 27/24, C08J 7/02, C08L 89/06, A61K 31/337

(54) **METHOD FOR REMOVING ORGANIC SOLVENT**

(30) Priority: 21.11.2006 JP 2006313956; 09.02.2007 JP 2007030123; 09.02.2007 JP 2007030124
(62) Divisional of application: 07832307.8
(71) Applicant: Fujifilm Corporation, Minato-ku Tokyo 106-8620 (JP)
(72) Inventor: Ooya, Shouji, Kanagawa, 258-8538 (JP); Hiratou, Tetsuo, Kanagawa, 258-8538 (JP)
(74) Representative: Hoffmann Eitle

(57) **Abstract**

The present invention is directed to a method for removing an organic solvent contained in a biopolymer structure from the structure, wherein the structure contains a hydrophilic compound.

## Description

### Technical Field

The present invention relates to a method for removing a residual organic solvent contained in a structure, and a structure comprising gelatin or collagen and containing a reduced content of a residual organic fluorine compound.

### Background Art

The use of synthetic polymers and biopolymers has been discussed in the field of cutting-edge medicine such as regenerative medicine that has recently made significant progress. Such polymer materials are mainly dissolved in a variety of solvents and subjected to molding/processing. When such materials are used for medical purposes, it is necessary to remove harmful solvents therefrom. In general, organic solvents are removed from such materials by vacuum drying, heating, or the combined use thereof.

Proteins such as collagen and gelatin can be dissolved using organic fluorine compounds represented by 1,1,1,3,3,3-hexafluoro-2-propanol (HFIP) and trifluoroethanol (TFE). JP Patent Publication (Kohyo) No. 2002-531182 A describes that a matrix for tissue formation can be produced by dissolving such a protein in HFIP, followed by molding.

Meanwhile, in recent years, electrospinning has been gaining attention as a technique for readily producing submicron-scale fibers. In the case of such technique, fibers are formed by injecting a polymer solution while applying a voltage to the solution. The fiber thickness depends on applied voltage, solution concentration, and the distances that sprayed particles move. A thin film having a three-dimensional structure (three-dimensional mesh structure) can be obtained by continuously forming fibers on a substrate. In addition, it is possible to form a film having a fabric-like thickness so as to produce a non-woven fabric with a submicron-scale mesh structure. Applied use of such non-woven fabric for spacesuits and protective suits is being studied. The above technique is used for formation of a structure used in the field of medicine (JP Patent Publication (Kohyo) No. 2004-532802 A; and JP Patent Publication (Kokai) No. 2004-321484 A). In particular, HFIP is mainly used as a solvent for producing a fibrous structure comprising collagen or gelatin by electrospinning. Since HFIP and TFE cause irritation and have other toxic properties, it is necessary to remove such solvents after production of structures particularly when using structures for medical purposes.

A structure produced with the above organic fluorine compound is subjected to air drying or vacuum drying. It has been reported that HFIP can be removed by vacuum drying (JP Patent Publication (Kohyo) No. 2002-531182 A). However, quantification of a residual solvent and intensive removal of such solvent have not been examined. When removal of HFIP from a material obtained by the above technique is insufficient, toxicity of HFIP remaining in a medical material might be observed. In addition, JP Patent Publication (Kohyo) No. 2002-531182 A, JP Patent Publication (Kokai) No. 2002-27283 A, and the like describe an operation of washing a structure (prepared with the use of HFIP) with water. However, in such case, the operation is aimed to remove salts and proteins but not HFIP contained in a structure.
Patent Document 1: JP Patent Publication (Kohyo) No. 2002-531182 A
Patent Document 2: JP Patent Publication (Kohyo) No. 2004-532802 A
Patent Document 3: JP Patent Publication (Kokai) No. 2004-321484 A
Patent Document 4: JP Patent Publication (Kokai) No. 2002-27283 A

### Disclosure of the Invention

### Object to be Solved by the Invention

It is an object of the present invention to provide a method for efficiently removing a residual organic solvent in a biopolymer structure which was a problem of conventional techniques.

### Means for Solving the Object

As a result of intensive studies in order to achieve the above object, the present inventors have found that it is possible to efficiently remove a residual solvent by use of an atmosphere containing a different solvent in a gas form instead of a general solvent removal means involving vacuum or high-temperature drying. Further, the present inventors have found that it is possible to remove a residual organic solvent in a biopolymer structure by washing the biopolymer structure with water. Furthermore, the present inventors have found that it is possible to efficiently remove a residual solvent by mixing a hydrophilic compound into a structure instead of using a general solvent removal means involving vacuum or high-temperature drying. That is, as described above, it has become possible to provide a method for efficiently removing a toxic organic solvent.

The first aspect of the present invention provides a method for removing an organic solvent contained in a biopolymer structure from the structure, which comprises placing the structure in an atmosphere containing a solvent other than the organic solvent so as to remove the organic solvent.

Preferably, the biopolymer is a protein.

Preferably, the protein is at least one selected from the group consisting of collagen, gelatin, albumin, laminin, casein, fibroin, fibrin, fibronectin, and vitronectin.

Preferably, the biopolymer is crosslinked.

Preferably, the solvent other than the organic solvent is a solvent compatible with the organic solvent.

Preferably, the solvent compatible with the organic solvent is water, alcohol, or ketone.

Preferably, the total vapor pressure of the solvent other than the organic solvent accounts for 55% or more of the saturated vapor pressure.

Preferably, the solvent other than the organic solvent is water, and the organic solvent is removed in an atmosphere with a humidity of 55% or more.

Preferably, the temperature at which the organic solvent is removed is 25°C to 200°C.

Preferably, the organic solvent to be removed is an organic fluorine compound.

Preferably, the organic fluorine compound is trifluoroethanol, 1,1,1,3,3,3-hexafluoro-2-propanol, hexafluoroacetone, trifluoroacetic acid, or pentafluoropropionic acid.

Another aspect of the present invention provides a composition comprising gelatin or collagen, which is composed of a structure comprising gelatin or collagen, provided that the structure is prepared by use of an organic fluorine compound and the residual organic fluorine compound content in the structure is 0.1% or less.

Preferably, the structure has a thickness of 1 nm or more.

Preferably, the organic fluorine compound is trifluoroethanol, 1,1,1,3,3,3-hexafluoro-2-propanol, hexafluoroacetone, trifluoroacetic acid, or pentafluoropropionic acid.

The second aspect of the present invention provides a method for removing an organic solvent contained in a biopolymer structure from the structure, wherein the organic solvent is removed by washing the structure with a solution mainly containing water.

Preferably, the biopolymer is a protein, a polysaccharide, or a derivative thereof.

Preferably, the biopolymer is a protein, or a derivative thereof.

Preferably, the biopolymer is a protein.

Preferably, the protein is at least one selected from the group consisting of collagen, gelatin, albumin, laminin, casein, fibroin, fibrin, fibronectin, and vitronectin.

Preferably, the protein is a human-, bovine-, pig-, or fish-derived protein or a gene recombinant protein.

Preferably, the polysaccharide is chitin, chitosan, hyaluronic acid, heparin, heparan sulfate, or chondroitin sulfate.

Preferably, the biopolymer is crosslinked.

Preferably, the biopolymer is crosslinked by heat or light, or with a condensation agent or an enzyme.

Preferably, the organic solvent to be removed is an organic fluorine compound.

Preferably, the organic fluorine compound is 1,1,1,3,3,3-hexafluoro-2-propanol, trifluoroethanol, hexafluoroacetone, trifluoroacetic acid, or pentafluoropropionic acid.

The third aspect of the present invention provides a method for removing an organic solvent contained in a biopolymer structure from the structure, wherein the structure contains a hydrophilic compound.

Preferably, the biopolymer is a protein.

Preferably, the protein is at least one selected from the group consisting of collagen, gelatin, albumin, laminin, casein, fibroin, fibrin, fibronectin, and vitronectin.

Preferably, the biopolymer is crosslinked.

Preferably, the water solubility of the hydrophilic compound is 1 mg/mL or more.

Preferably, the boiling point of the hydrophilic compound is 100°C or more.

Preferably, the hydrophilic compound is sugars, salts, alcohols, carboxylic acids, ethers, amines, or amides.

Preferably, the hydrophilic compound is glycerol, ethylene glycol, propylene glycol, butylene glycol, polyethylene glycol, sodium chloride, lactose, sodium poiyphosphate, or sodium L-ascorbate.

Preferably, the organic solvent to be removed is an organic fluorine compound.

Preferably, the organic fluorine compound is trifluoroethanol, 1,1,1,3,3,3-hexafluoro-2-propanol, hexafluoroacetone, trifluoroacetic acid, or pentafluoropropionic acid.

Preferably, the organic fluorine compound is trifluoroethanol or 1,1,1,3,3,3-hexafluoro-2-propanol.

### Effect of the Invention

According to the method of the present invention, it is possible to efficiently remove a residual solvent contained in a structure and used in production steps. According to the method the present invention, unlike a method wherein impurities are removed from a structure by immersing the structure in a solvent, it is possible to remove a residual solvent that is a good solvent for an inclusion compound while minimizing deformation of a structure due to elution or swelling in a solvent and preventing effusion of an inclusion compound.

### Best Mode for Carrying Out the Invention

In recent years, structures used for artificial organs or artificial tissues that are produced in the field of advanced medicine have been implanted *in vivo* for use. Thus, such a structure is required to have higher purity than materials used for non-medical purposes. That is, it is necessary to completely remove impurities such as solvents used in production steps or to reduce the amount of such impurities to safe levels.

In the 1^{st} embodiment of the present invention, a method for removing an organic solvent contained in a biopolymer structure from the structure comprises the step of placing the structure in an atmosphere containing a solvent other than the organic solvent so as to remove the organic solvent.

There are many benchmarks for selection of a solvent used in a gas form. Examples of benchmarks include boiling point, affinity to a structure containing a residual solvent, safety, and presence of a compound (drug or pigment) in a structure. The boiling points of a solvent used in a gas form and a residual solvent are not particularly defined. However, it is preferable that the boiling point of the former be higher than that of the latter (a residual solvent). In addition, regarding another benchmark for production of a structure that can be used *in vivo*, the solvent used in a gas form is preferably water, alcohol, or ketone, more preferably water or alcohol, further preferably water, ethanol, or isopropanol, even more preferably water or ethanol, and most preferably water. In addition, a single solvent may be used in a gas form. Alternatively, a gas mixture of at least two different solvents may be used. Further, preferably, the capacity of dissolving or swelling of the above structure is used as another benchmark for selection of a solvent used in a gas form.

The total vapor pressure of a solvent in a gas form at the above temperature accounts for preferably 55% or more, more preferably 70% or more, and most preferably 80% or more of the saturated vapor pressure. However, it is not particularly limited thereto. For instance, if a solvent in a gas form is water, humidity is represented by the solvent vapor pressure that accounts for a certain percentage of the saturated vapor pressure. Specifically, if a solvent that differs from an organic solvent is water, the humidity is preferably 55% or more, more preferably 70% or more, and most preferably 80% or more. In addition, the saturated water vapor volume significantly varies in a temperature-dependent manner. Thus, the humidity that is necessary at a temperature at which the solvent is removed might vary. In addition, the gas temperature is not particularly limited. However, it is preferably from 25°C to 200°C, more preferably from 30°C to 100°C, and most preferably from 35°C to 80°C. Temperatures in the system would significantly vary depending on types of residual solvents and substances mixed therewith. However, preferably, the temperature is slightly below the boiling point of a residual solvent.

In the 2^{nd} embodiment of the method of the present invention, the method comprises the step of removing an organic solvent by washing a structure with a solution mainly containing water. A solution mainly containing water used for washing may be pure water or an aqueous solution containing various additives. Examples of a compound that can be added to an aqueous solution include various inorganic salts, pH adjusters, and solvents compatible with water. More preferably, an aqueous solution is deionized water or an aqueous solution containing various inorganic salts. Most preferably, an aqueous solution is deionized water or a buffer solution. Washing operations are not particularly defined. However, it is possible to immerse a structure in a large amount of water or an aqueous solution or to spray water or an aqueous solution onto a structure. Water or an aqueous solution can be used for washing as long as the temperature thereof is a temperature at which water can exist in a liquid form. Such a temperature is preferably from 0°C to 60°C, more preferably from 0°C to 40°C, and most preferably from 0°C to 30°C.

The pH of a solution is not particularly limited as long as the present invention can be carried out. However, since a biopolymer is used, the pH is preferably approximately neutral. The pH is preferably 5 to 10 and more preferably 6 to 9. In another embodiment, the optimum pH of a washing solvent varies depending on the acidity of a residual organic solvent. For instance, a weak alkaline pH (7 < pH < 9) is preferable for a highly acidic solvent (pKa < 15.7). In addition, a weak acidic pH (5 < pH < 7) is preferable for a highly basic solvent (pKa > 15.7).

In the 3^{rd} embodiment of the present invention, an organic solvent contained in a biopolymer structure can be removed from the structure by drying the structure. The vapor pressure of a solvent in a gas form upon drying accounts for preferably 55% or more, more preferably 70% or more, and most preferably 80% or more of the saturated vapor pressure. However, it is not particularly limited thereto. Thus, when the percentage is increased, the solvent removal rate can be improved. For instance, if a solvent in a gas form is water, humidity is represented by the solvent vapor pressure that accounts for a certain percentage of the saturated vapor pressure. Specifically, if a solvent that differs from an organic solvent is water, the humidity is preferably 55% or more, more preferably 70% or more, and most preferably 80% or more. In addition, the saturated water vapor volume significantly varies in a temperature-dependent manner. Thus, the humidity that is necessary at a temperature at which such a solvent is removed might vary. In addition, the gas temperature is not particularly limited. However, it is preferably from 25°C to 200°C, more preferably from 30°C to 100°C, and most preferably from 35°C to 80°C. Temperatures in the system would significantly vary depending on types of residual solvents and substances mixed therewith. However, preferably, the temperature is slightly below the boiling point of a residual solvent. In addition, the pressure upon drying is not particularly limited. Drying at normal pressures, drying by pressurization, or vacuum drying can be carried out. Further, air blowing may be carried out.

In the 3^{rd} embodiment of the present invention, the structure comprises a hydrophilic compound. The water solubility of the hydrophilic compound that can be used in the present invention is preferably 1 mg/mL or more and further preferably from 1 mg/mL to 200 mg/mL. The boiling point of the hydrophilic compound is preferably 100°C or more and further preferably from 100°C to 1500°C. The degree of hygroscopicity of the hydrophilic compound (the amount of saturated water absorption per unit weight) is preferably 1 mg/g or more and further preferably from 10 mg/g to 1000 g/g.

The above hydrophilic compound is not particularly limited as long as the present invention can be carried out. Low-molecular polymers, synthetic polymers, or biopolymers may be used. Preferred examples thereof include glycerol, ethylene glycol, propylene glycol, butylene glycol, polyethylene glycol, sodium chloride, lactose, sodium polyphosphate, and sodium L-ascorbate. In another embodiment, a hydrophilic compound may be hygroscopic. Further, in another embodiment, a hydrophilic compound may be water-insoluble. For instance, the structure may contain water-insoluble particles obtained by treating a water-insoluble compound (e.g., a metal or a hydrophobic polymer) in a manner such that particles of the compound have hydrophilic or hygroscopic surfaces.

The content of a hydrophilic compound in a biopolymer structure is not particularly limited as long as effects of the present invention can be obtained. However, the content is generally from 0.001% by weight to 10% by weight, preferably from 0.1% by weight to 10% by weight, and further preferably from 0.5% by weight to 5% by weight.

Herein, a biopolymer that is a biologically derived polymer is not particularly limited. However, preferred examples thereof include a protein, a sugar, a polysaccharide, and a derivative or salt of either thereof. For example, when a protein is used, any protein in a spherical form or a fibrous form can be used. Preferred examples of a biopolymer include: collagen, gelatin, albumin, laminin, casein, fibroin, fibrin, fibronectin, vitronectin, and derivatives of any one of the above examples; hyaluronic acid; and hyaluronic acid ester (hyaluronate). More preferably, collagen, gelatin, albumin, casein, or fibroin is used. Most preferably, collagen or gelatin is used. The protein origin is not particularly limited. Any human-, bovine-, pig-, or fish-derived proteins or gene recombinants proteins can be used. Examples of gene recombinant gelatins that can be used are those described in EU1014176A2, US6992172, or the like; however, it is not limited thereto.

The form of a biopolymer is not particularly limited. However, it can be formed into a non-crosslinked product, a physically or chemically crosslinked product, a chemically modified product, or a mixture of such products. In addition, a structure does not necessarily consist of a biopolymer, and thus a structure may partly contain a biopolymer.

In a case in which a structure is a crosslinked biopolymer product, crosslinking can be carried out using heat, light, a crosslinking agent (condensation agent), or an enzyme. By controlling the degree of crosslinking for a biopolymer, a structure can obtain different properties such as biodegradability, strength, and structural properties. A crosslinking method is not particularly limited. Examples of a crosslinking method include physical crosslinking, chemical crosslinking, thermal crosslinking, and enzymatic crosslinking. Preferably, chemical or enzymatic crosslinking is carried out. Examples of a chemical crosslinking agent include aldehydes such as glutaraldehyde and formaldehyde, carbodiimide, and cyanamide, which have been widely used in general. More preferably, enzymatic crosslinking is carried out.

In a case in which enzymatic crosslinking is carried out, an enzyme used is not particularly limited as long as it has an action of crosslinking a protein. However, crosslinking can be carried out preferably using transglutaminase and laccase and most preferably using transglutaminase. Examples of proteins that are enzymatically crosslinked by transglutaminase are not particularly limited, so long as the proteins have lysine residues and glutamine residues. A mammalian-derived or microorganism-derived transglutaminase may be used. Specific examples thereof include: the Activa series (produced by Ajinomoto Co., Inc.); commercially available mammalian-derived transglutaminases serving as reagents such as guinea pig liver-derived transglutaminase, goat-derived transglutaminase, and rabbit-derived transglutaminase (produced by Oriental Yeast Co., Ltd., Upstate USA Inc., Biodesign International, etc.); and a human-derived blood coagulation factor (Factor XIIIa, Haematologic Technologies, Inc.).

A residual organic solvent is not particularly defined. However, it is preferably a water compatible solvent. It is preferably an organic fluorine compound, more preferably an organic fluorine compound having a carbon number of 1 to 8, further preferably an organic fluorine compound having a carbon number of 1 to 6, and even more preferably an organic fluorine compound having a carbon number of 1 to 3. Further preferably, such an organic fluorine compound is alcohol, ketone, or carboxylic acid. Particularly preferably, it is 1,1,1,3,3,3-hexafluoro-2-propanol, trifluoroethanol, hexafluoroacetone, trifluoroacetic acid, or pentafluoropropionic acid. Most preferably, it is 1,1,1,3,3,3-hexafluoro-2-propanol or trifluoroethanol. Further, it may consist of not only a single residual organic solvent but also at least 2 types of organic solvents.

In addition, an organic fluorine compound is not particularly limited as long as it is an organic compound containing fluorine. However, examples thereof include fluorine-containing alcohols, fluorine-containing amides, fluorine-containing esters, fluorine-containing carboxylic acids, fluorine-containing ethers, fluorine-containing nitriles, fluorine-containing chlorides, and fluorine-containing bromides. Further, such an organic fluorine compound may be an aliphatic, aromatic, saturated, or unsaturated compound.

Next, the method for producing a biopolymer structure of the present invention is described. The method for producing a biopolymer structure is not particularly limited as long as an organic solvent is used in the method. For instance, such a biopolymer structure can be produced by application and drying of a mixture obtained by dissolving a biopolymer in an organic solvent such as an organic fluorine compound. For example, a film can be formed by applying a mixture obtained by dissolving a drug and a biopolymer in an organic solvent such as an organic fluorine compound to a substrate, followed by drying.

In a case in which an organic fluorine compound is used to produce a protein structure, it is problematic for the compound to contain a residual organic fluorine compound when used for medical purposes. The residual organic fluorine content in the structure is preferably 1% or less, more preferably 0.1% or less, and most preferably 0.01% or less.

The form of the structure is not particularly limited. The structure may be formed into gel, sponge, film, non-woven fabric, fibers (tubes), particles, or the like. The structure can be used in any form. However, a pyramidal, conical, rectangular cylindrical, circular cylindrical, spherical, or spindle-shaped structure or a structure produced using a mold with a desired shape can be used. Preferably, a rectangular cylindrical, circular cylindrical, or spindle-shaped structure or a structure produced using a mold with a desired shape can be used. More preferably, a pyramidal, conical, rectangular cylindrical, or circular cylindrical structure can be used. Most preferably, a rectangular cylindrical or circular cylindrical structure can be used. The size of the structure is not particularly limited. However, when the structure is in the form of gel, sponge, or non-woven fabric, the size thereof is preferably 500 centimeters square or less, preferably 100 centimeters square or less, particularly preferably 50 centimeters square or less, and most preferably 10 centimeters square or less. When it is formed into a fiber (tube), the diameter of a fiber or tube (or one side of the cross section thereof) is from 1 nm to 10 cm, preferably from 1 nm to 1 cm, more preferably from 1 nm to 100 µm, particularly preferably from 1 nm to 1 µm, and most preferably from 1 nm to 10 nm. In addition, the length thereof is not particularly limited. However, the length is preferably from 10 µm to 100 m, more preferably from 100 µm to 10 m, further preferably from 1 mm to 1 m, and most preferably from 1 cm to 30 cm. When the composition is formed into particles, the particle size is preferably from 1 nm to 1 mm, more preferably from 10 nm to 200 µm, further preferably from 50 nm to 100 µm, and particularly preferably from 100 nm to 10 µm.

The thickness of a structure is not particularly limited. However, the thickness is preferably 1 nm or more, more preferably 10 nm or more, further preferably 100 nm or more, even more preferably 1 µm or more, yet more preferably 10 µm or more, and most preferably 100 µm or more.

It is possible to add an additive to the above composition according to need. Examples of additives include drugs, pigments, softening agents, transdermal-absorption-promoting agents, moisturizing agents, surfactants, preservatives, aroma chemicals, and pH adjusters.

Specific examples of drugs include anticancer agents (e.g., paclitaxel, Topotecin, taxotere, and 5-fluorouracil), immunosuppressive agents (e.g., Rapamycin, tacrolimus, and cyclosporine), anti-inflammatory agent, antithrombotic agents, antipsychotic agents, antidepressants, antioxidants, antiallergic agents, growth factors, hormones, supplement components, and cosmetic components.

Applications of the above structure are not particularly limited. However, it can be used for a transdermally absorbable agent, a topical therapeutic agent, an implantable agent, an oral therapeutic agent, a cosmetic, a supplement, a food, and a pigment. Preferably, it can be used for a transdermally absorbable agent, a topical therapeutic agent, an oral therapeutic agent, and a cosmetic. Further preferably, it can be used for a transdermally absorbable agent, a topical therapeutic agent, an implantable agent, and an oral therapeutic agent. Most preferably, it can be used for a transdermally absorbable agent and a topical therapeutic agent.

### Examples

The present invention is hereafter described in greater detail with reference to the following examples, although the present invention is not limited thereto. In this Example, a case in which an organic solvent was removed using water which is the most preferable solvent in terms of use for medical materials, is described.

### Example 1: Removal of 1,1,1,3,3,3-hexafluoro-2-propanol (HFIP) from a gelatin film

An HFIP solution containing acid-treated gelatin (20%; PSP gelatin produced by Nippi Inc.) and paclitaxel (1 mg/mL) was coated onto a polypropylene substrate (coating thickness: 1 mm). The obtained film was allowed to stand under different solvent removal conditions or subjected to air drying or vacuum drying. Thus, paclitaxel-containing gelatin films were obtained. The obtained gelatin films were immersed overnight in methanol for extraction of residual HFIP. The HFIP content was quantified by GCMS (GCMS-QP2010, produced by Shimadzu Corporation; column: DB-624; 60 m; ϕ = 0.25 mm).

Separately, a gelatin film containing paclitaxel mixed with 1% glycerine was prepared in a similar manner, followed by drying at 60°C (humidity: 95%). Then, the HFIP residual content was quantified in a similar manner.

In a case in which the paclitaxel-containing gelatin film was subjected to air drying, the residual HFIP content was 25.7%. In a case in which the film was subjected to vacuum drying at 50°C, the residual HFIP content was 18%, which was lower than the content in the case of air drying. Meanwhile, in a case in which solvent removal was carried out by adding water to an atmosphere to a humidity of 80%, the residual HFIP content significantly decreased. 72 hours later, it decreased to 0.2%. 168 hours later, it decreased to 0.002%. Further, in a case in which drying was carried out at 50°C with a humidity of 95% or at 60°C with a humidity of 80%, the content decreased to 0.001% or less 72 hours later. It was possible to reduce the residual HFIP content to a level equivalent to approximately 1/10000 of that in a sample subjected to vacuum drying for the same period of time by increasing the humidity in the system. It is possible to say that the HFIP content was successfully reduced with good efficiency with the addition of a large amount of water vapor to an atmosphere upon removal of HFIP.

In addition, the above operation allowed removal of HFIP from an albumin film (produced from a 10% solution) in a similar manner.
[Table 1]

**Table 1: The residual HFIP content obtained under different residual solvent removal conditions**

| Pressure (mmHg) | Time (hour) | Temperature (°C) | Humidity (%) | Residual HFIP content (%) |
|---|---|---|---|---|
| 1 | 72 | 50 | - | 17.9 * |
| 760 | 48 | 25 | 50 | 25.7 |
| 760 | 5 | 50 | 50 | 24.1 * |
| 760 | 72 | 40 | 80 | 4.1 ** |
| 760 | 168 | 40 | 80 | 0.1 ** |
| 760 | 72 | 50 | 80 | 0.2 ** |
| 760 | 168 | 50 | 80 | 0.002** |
| 760 | 72 | 50 | 95 | < 0.001** |
| 760 | 72 | 60 | 80 | < 0.001** |
| 760 | 72 | 60 | 95 | < 0.001** |
| 760 | 72 | 60 | 95 | < 0.001**^{Note 1} |
| 760 | 168 | 60 | 95 | < 0.001**^{Note 2} |

| | | | | |
|---|---|---|---|---|
| *: Drying at room temperature (humidity: 50%) for 15 hours; **: Air drying for 3 days after recovery of a sample Note: 1: Film containing glycerol (1%); Note 2: Albumin film | | | | |

### Example 2: Stability of paclitaxel contained in a film

The paclitaxel-containing gelatin film prepared in Example 1 was treated with Actinase to degrade gelatin, followed by extraction with ethyl acetate. Thus, paclitaxel was quantitatively recovered. The recovered paclitaxel was analyzed by HPLC (Tosoh Corporation, TSK-gel ODS-80Ts; eluent: THF/water = 9/1). Paclitaxel was found not to be degraded and thus contained in the gelatin film. In addition, upon removal of HFIP, which is a good solvent for paclitaxel, it was possible to prevent effusion of paclitaxel from the gelatin film. Further, a gelatin film prepared with HFIP has been problematic because of warpage of film edges upon drying. However, it was possible to improve such warpage of a film with the use of the present invention.

### Example 3: Removal of 1,1,1,3,3,3-hexafluoro-2-propanol (HFIP) from a gelatin film

An HFIP solution containing acid-treated gelatin (10% or 20%; PSP gelatin, produced by Nippi Inc.) and paclitaxel (1 mg/mL) was coated onto a polypropylene substrate (size: 20 cm x 20 cm; application thickness: 1 mm). The film was immersed in deionized water for 1 hour at 4°C or 20°C. After crosslinking and water washing operations, air drying was carried out for one day. Accordingly, a paclitaxel-containing gelatin film was obtained.

Separately, an HFIP solution containing the above acid-treated gelatin (10.4%) and paclitaxel (1.0 mg/mL) was mixed with a 25% glutaraldehyde aqueous solution (HFIP/water = 24), followed by stirring at 4°C (final concentration: gelatin 10%; paclitaxel: 1 mg/mL; and glutaraldehyde: 1%). The resultant was coated onto a polypropylene substrate (size: 20 cm x 20 cm; application thickness: 1 mm) and allowed to stand at room temperature for 15 hours. The thus obtained film was immersed in deionized water for 1 hour at 4°C or 20°C. After water washing operations, air drying was carried out for one day. Accordingly, a paclitaxel-containing crosslinked gelatin film was obtained.

The gelatin film was immersed overnight in methanol for extraction of residual HFIP. The HFIP content was quantified by GCMS (GCMS-QP2010, produced by Shimadzu Corporation; column: DB-624; 60 m; ϕ = 0.25 mm). Table 2 shows the results.

In a case in which the paclitaxel-containing gelatin film was subjected to air drying, the residual HFIP content was 17% to 19 % (gelatin: 10%) or 25.7% (gelatin: 20%). In a case in which the film (gelatin: 10%) was subjected to vacuum drying (1 mmHg) at 50°C, the residual HFIP content decreased to 19.2%, which was lower than the content obtained in the case of air drying. That is, even after air drying, the gelatin film still contained a large amount of HFIP. Also, it can be said that vacuum drying is less effective for removal of HFIP from a gelatin film.

Meanwhile, as a result of crosslinking treatment of the air-dried film followed by a water washing operation, the HFIP content in the film significantly decreased to 0.003% (gelatin: 10%) or 0.012% (gelatin: 20%). In addition, the HFIP content similarly decreased, also due to a water washing operation at 20°C (table 2). Thus, it can be said that usual vacuum drying is insufficient for removal of HFIP from a gelatin film, and that a water washing operation is effective for removal of HFIP from a gelatin structure.
[Table 2]

**Table 2: The residual HFIP contents under different residual solvent removal conditions**

| Gelatin concentration (%) | Water washing/ No water washing | Crosslinking | Temperature for water washing (°C) | Residual HFIP content (%) |
|---|---|---|---|---|
| 10 | None* | None | - | 17.9 |
| 10 | None** | None | - | 19.2 |
| 10 | Implemented | None | 4 | 0.003 |
| 10 | Implemented | None | 20 | 0.002 |
| 10 | None | Implemented | - | 18.8 |
| 10 | Implemented | Implemented | 4 | 0.008 |
| 10 | Implemented | Implemented | 20 | 0.005 |
| 20 | None* | None | - | 25.7 |
| 20 | Implemented | None | 4 | 0.012 |
| 20 | Implemented | None | 20 | 0.010 |

| | | | | |
|---|---|---|---|---|
| *: Drying at room temperature for 48 hours; **: Drying at room temperature for 15 hours and vacuum drying at 50°C for 3 days | | | | |

### Example 4: Removal of 1,1,1,3,3,3-hexafluoro-2-propanol (HFIP) from gelatin film

An HFIP solution containing acid-treated gelatin (20%; PSP gelatin produced by Nippi Inc.), paclitaxel (1 mg/mL), and a certain amount of glycerol or Activa TG-S (produced by Ajinomoto Co., Inc.; composition: transglutaminase: 1%; sodium polyphosphate: 5%; sodium pyrophosphate: 5%; L-sodium ascorbate: 0.5%; and lactose: 88.5%) was coated onto a polypropylene substrate. Thus, a film was prepared (application thickness: 1 mm). The film was allowed to stand under different solvent removal conditions, followed by air drying for 3 days. Thus, a paclitaxel-containing gelatin film was obtained. The gelatin film was immersed overnight in methanol for extraction of residual HFIP. The HFIP content was quantified by GCMS (GCMS-QP2010 produced by Shimadzu Corporation; column: DB-624; 60 m; ϕ = 0.25 mm).

In a case in which the paclitaxel-containing gelatin film free from glycerol was subjected to air drying, the residual HFIP content was approximately 25%, which was comparable to that in the case of the film free from paclitaxel. The presence of paclitaxel did not influence the HFIP removal efficiency. In a case in which the film was subjected to vacuum drying at 50°C, the residual HFIP content decreased to approximately 18%, which was lower than the content obtained in the case of air drying. Meanwhile, addition of glycerol to the film resulted in a significant decrease in the residual HFIP content, depending on the glycerol concentration. In such case, the residual HFIP content was 5.3% with the use of glycerol (5%) (humidity: 5%). In a case in which the humidity was increased to 70%, HFIP removal was more effectively carried out. Accordingly, the content decreased to 0.03% at a glycerol concentration of 5%. In a case in which the humidity was increased to 95%, the residual HFIP content in the film containing glycerol (1.0%) further decreased to 0.001% or less. Thus, the HFIP removal efficiency was improved when adding glycerol to a gelatin film prepared with HFIP. Therefore, it can be said that HFIP removal was carried out in a more efficient manner than in the case of usual vacuum drying. Also, it can be said that such removal effects can be improved by increasing the humidity.

Meanwhile, in a case in which a gelatin film comprising a mixture of different salts (such mixture mainly containing lactose) was dried (temperature: 50°C; and humidity: 80%), the residual HFIP content significantly decreased (residual HFIP: < 0.001%) to a level below that in a film free from additives (residual HFIP: 0.23%). It can be said that the residual HFIP content was significantly decreased using, as an additive, a mixture of different salts (mainly containing lactose).

Further, HFIP was successfully removed from an albumin film (produced from a 10% solution) by the above operations in a similar manner.
[Table 3]

**Table 3: The residual HFIP contents under different residual solvent removal conditions**

| Glycerol concentration (%) | Pressure (mmHg) | Time (hour) | Temperature (°C) | Humidity (%) | Residual HFIP content (%) |
|---|---|---|---|---|---|
| 0 | 760 | 72 | 25 | 50 | 25.6 ^{Note} |
| 0 | 760 | 72 | 25 | 50 | 25.7 |
| 0 | 1 | 72 | 50 | - | 17.9 * |
| 0 | 760 | 72 | 50 | 5 | 21.1 ** |
| 0 | 760 | 72 | 50 | 70 | 14.6 ** |
| 0 | 760 | 120 | 50 | 70 | 13.8 ** |
| 0 | 760 | 168 | 50 | 70 | 12.6 ** |
| 0*** | 760 | 72 | 50 | 80 | <0.001 |
| 0 | 760 | 72 | 50 | 80 | 0.23 |
| 0.5 | 760 | 72 | 50 | 5 | 18.6 ** |
| 0.5 | 760 | 72 | 50 | 70 | 7.6 ** |
| 1.0 | 760 | 72 | 50 | 5 | 18.4** |
| 1.0 | 760 | 72 | 50 | 70 | 2.3** |
| 1.0 | 760 | 168 | 50 | 70 | 0.3 |
| 1.0 | 760 | 24 | 50 | 95 | <0.001 |
| 5.0 | 760 | 72 | 50 | 5 | 5.3** |
| 5.0 | 760 | 72 | 50 | 70 | 0.03** |
| 5.0 | 760 | 72 | 50 | 70 | 0.03**** |

| | | | | | |
|---|---|---|---|---|---|
| Note: no paclitaxel *: After drying at room temperature (humidity: 50%) for 15 hours **: Air drying for 3 days after recovery of a sample ***: ActivaTG-S 1% ****: Albumin film (produced from a 10% solution) | | | | | |

### Example 5: Stability of paclitaxel contained in a film

The paclitaxel-containing gelatin film prepared in Example 4 was treated with Actinase to degrade gelatin, followed by extraction with ethyl acetate. Thus, paclitaxel was quantitatively recovered. The recovered paclitaxel was analyzed by HPLC (Tosoh Corporation; TSK-gel ODS-80Ts; eluent: THF/water = 9/1). Paclitaxel was found not to be degraded and thus to be contained in the gelatin film. In addition, when HFIP, which is a good solvent for paclitaxel, was removed, it was possible to prevent effusion of paclitaxel from the gelatin film. Further, a gelatin film prepared with HFIP has been problematic because of warpage of film edges upon drying. However, it was possible to improve such warpage of a film with the use of the present invention.

### Example 6: Removal of HFIP from a paclitaxel-containing gelatin sponge

A PBS solution containing gelatin (PSK gelatin produced by Nippi Inc.) and glutaraldehyde (GA) (final concentration: gelatin: 10%; GA: 0.1%; and total volume: 20 mL) was poured into a container (5 x 10 cm x 4 mm) and allowed to stand at 4°C for 17 hours. Thus, a GA crosslinked gelatin gel was prepared (thickness: 4 mm). The gel was immersed in a 50 mM glycine solution (100 mL) at 37°C and allowed to stand for 1 hour. Further, the gel was immersed (twice) in deionized water (100 mL) at 37°C and allowed to stand for 1 hour. The obtained gel was perforated (ϕ = 12 mm), followed by lyophilization. Thus, a gelatin sponge was obtained. An HFIP solution containing paclitaxel (paclitaxel concentration: 1.5% or 0.7%; volume: 50 µL or 100 µL) was added to the sponge at room temperature such that the gelatin sponge became swollen and paclitaxel was allowed to permeate the gelatin sponge. The obtained paclitaxel-containing gelatin sponge was allowed to stand under certain conditions (temperature: 50°C; humidity: 95%) for 3 days, followed by air drying for one day. Thus, a paclitaxel-containing gelatin sponge was obtained. The sponge was immersed in a 0.4% Actinase aqueous solution (1.5 mL) and Actinase was allowed to act overnight at 40°C to dissolve gelatin. The thus obtained aqueous solution was introduced into GC (GC-2010; produced by Shimadzu Corporation; column: RTx-Stabilwax 30 m; ϕ = 0.32). The HFIP content in the gelatin was quantified by quantifying the HFIP content in the solution. The HFIP content in the sponge was 0.001% or less (9.3% 4 days after air drying) in each case. Based on the results, it can be said that HFIP can be removed from gelatin even when such gelatin is in a sponge form.

### Industrial Applicability

According to the method of the present invention, a residual solvent used during production of a structure and contained in the structure can be efficiently removed therefrom.

The present invention is related to the following aspects:
1. A method for removing an organic solvent contained in a biopolymer structure from the structure, which comprises placing the structure in an atmosphere containing a solvent other than the organic solvent so as to remove the organic solvent.
2. The method for removing an organic solvent according to aspect 1, wherein the biopolymer is a protein.
3. The method for removing an organic solvent according to aspect 2, wherein the protein is at least one selected from the group consisting of collagen, gelatin, albumin, laminin, casein, fibroin, fibrin, fibronectin, and vitronectin.
4. The method for removing an organic solvent according to any one of aspects 1 to 3, wherein the biopolymer is crosslinked.
5. The method for removing an organic solvent according to any one of aspects 1 to 4, wherein the solvent other than the organic solvent is a solvent compatible with the organic solvent.
6. The method for removing an organic solvent according to aspect 5, wherein the solvent compatible with the organic solvent is water, alcohol, or ketone.
7. The method for removing an organic solvent according to any one of aspects 1 to 6, wherein the total vapor pressure of the solvent other than the organic solvent accounts for 55% or more of the saturated vapor pressure.
8. The method for removing an organic solvent according to any one of aspects 1 to 7, wherein the solvent other than the organic solvent is water and the organic solvent is removed in an atmosphere with a humidity of 55% or more.
9. The method for removing an organic solvent according to any one of aspects 1 to 8, wherein the temperature at which the organic solvent is removed is 25°C to 200°C.
10. The method for removing an organic solvent according to any one of aspects 1 to 9, wherein the organic solvent to be removed is an organic fluorine compound.
11. The method for removing an organic solvent according to aspect 10, wherein the organic fluorine compound is trifluoroethanol, 1,1,1,3,3,3-hexafluoro-2-propanol, hexafluoroacetone, trifluoroacetic acid, or pentafluoropropionic acid.
12. A composition comprising gelatin or collagen, which is composed of a structure comprising gelatin or collagen, provided that the structure is prepared by use of an organic fluorine compound and the residual organic fluorine compound content in the structure is 0.1 % or less.
13. The composition comprising gelatin or collagen according to aspect 12, wherein the structure has a thickness of 1 nm or more.
14. The composition comprising gelatin or collagen according to aspect 12 or 13, wherein the organic fluorine compound is trifluoroethanol, 1,1,1,3,3,3-hexafluoro-2-propanol, hexafluoroacetone, trifluoroacetic acid, or pentafluoropropionic acid.
15. A method for removing an organic solvent contained in a biopolymer structure from the structure, wherein the organic solvent is removed by washing the structure with a solution mainly containing water.
16. The method for removing an organic solvent according to aspect 15, wherein the biopolymer is a protein.
17. The method for removing an organic solvent according to aspect 2, wherein the protein is at least one selected from the group consisting of collagen, gelatin, albumin, laminin, casein, fibroin, uorin, uoronectiu, and vitronectin.
18. The method for removing an organic solvent according to any one of aspects 15 to 17, wherein the protein is a human-, bovine-, pig-, or fish-derived protein or a gene recombinant protein.
19. The method for removing an organic solvent according to any one of aspects 15 to 18, wherein the biopolymer is crosslinked.
20. The method for removing an organic solvent according to aspect 19, wherein the biopolymer is crosslinked by heat or light, or with a condensation agent or an enzyme.
21. The method for removing an organic solvent according to any one of aspects 15 to 20, wherein the organic solvent to be removed is an organic fluorine compound.
22. The method for removing an organic solvent according to aspect 21, wherein the organic fluorine compound is 1,1,1,3,3,3-hexafluoro-2-propanol, trifluoroethanol, hexafluoroacetone, trifluoroacetic acid, or pentafluoropropionic acid.
23. A method for removing an organic solvent contained in a biopolymer structure from the structure, wherein the structure contains a hydrophilic compound.
24. The method for removing an organic solvent according to aspect 23, wherein the biopolymer is a protein.
25. The method for removing an organic solvent according to aspect 24, wherein the protein is at least one selected from the group consisting of collagen, gelatin, albumin, laminin, casein, fibroin, fibrin, fibronectin, and vitronectin.
26. The method for removing an organic solvent according to any one of aspects 23 to 25, wherein the biopolymer is crosslinked.
27. The method for removing an organic solvent according to any one of aspects 23 to 26, wherein the water solubility of the hydrophilic compound is 1 mg/mL or more.
28. The method for removing an organic solvent according to any one of aspects 23 to 27, wherein the boiling point of the hydrophilic compound is 100°C or more.
29. The method for removing an organic solvent according to any one of aspects 23 to 28, wherein the hydrophilic compound is hygroscopic.
30. The method for removing an organic solvent according to any one of aspects 23 to 26, wherein the hydrophilic compound is glycerol, ethylene glycol, propylene glycol, butylene glycol, polyethylene glycol, sodium chloride, lactose, sodium polyphosphate, or sodium L-ascorbate.
31. The method for removing an organic solvent according to any one of aspects 23 to 30, wherein the organic solvent to be removed is an organic fluorine compound.
32. The method for removing an organic solvent according to aspect 31, wherein the organic fluorine compound is trifluoroethanol, 1,1,1,3,3,3-hexafluoro-2-propanol, hexafluoroacetone, trifluoroacetic acid, or pentafluoropropionic acid.
33. The method for removing an organic solvent according to aspect 32, wherein the organic fluorine compound is trifluoroethanol or 1,1,1,3,3,3-hexafluoro-2-propanol.

## Claims

1. A method for removing an organic solvent contained in a biopolymer structure from the structure, wherein the structure contains a hydrophilic compound.

2. The method for removing an organic solvent according to claim 1, wherein the biopolymer is a protein.

3. The method for removing an organic solvent according to claim 2, wherein the protein is at least one selected from the group consisting of collagen, gelatin, albumin, laminin, casein, fibroin, fibrin, fibronectin, and vitronectin.

4. The method for removing an organic solvent according to any one of claims 1 to 3, wherein the biopolymer is crosslinked.

5. The method for removing an organic solvent according to any one of claims 1 to 4, wherein the water solubility of the hydrophilic compound is 1 mg/mL or more.

6. The method for removing an organic solvent according to any one of claims 1 to 5, wherein the boiling point of the hydrophilic compound is 100°C or more.

7. The method for removing an organic solvent according to any one of claims 1 to 6, wherein the hydrophilic compound is hygroscopic.

8. The method for removing an organic solvent according to any one of claims 1 to 4, wherein the hydrophilic compound is glycerol, ethylene glycol, propylene glycol, butylene glycol, polyethylene glycol, sodium chloride, lactose, sodium polyphosphate, or sodium L-ascorbate.

9. The method for removing an organic solvent according to claim 8, wherein the hydrophilic compound is glycerol, ethylene glycol, propylene glycol, butylene glycol, or polyethylene glycol.

10. The method for removing an organic solvent according to claim 8 or 9, wherein the hydrophilic compound is glycerol.

11. The method for removing an organic solvent according to any one of claims 1 to 10, wherein the organic solvent to be removed is an organic fluorine compound.

12. The method for removing an organic solvent according to claim 11, wherein the organic fluorine compound is trifluoroethanol, 1,1,1,3,3,3-hexafluoro-2-propanol, hexafluoroacetone, trifluoroacetic acid, or pentafluoropropionic acid.

13. The method for removing an organic solvent according to claim 12, wherein the organic fluorine compound is trifluoroethanol or 1,1,1,3,3,3-hexafluoro-2-propanol.

14. The method for removing an organic solvent according to any one of claims 1 to 13, wherein the organic solvent is removed at a temperature of 35°C to 80°C.
